(19) **Europäisches Patentamt / European Patent Office / Office européen des brevets**

(11) **EP 4 249 030 A1**

(12) # EUROPEAN PATENT APPLICATION
published in accordance with Art. 153(4) EPC

(43) Date of publication:
**27.09.2023 Bulletin 2023/39**

(21) Application number: **21905465.7**

(22) Date of filing: **24.11.2021**

(51) International Patent Classification (IPC):
***A61M 25/00*** (2006.01)

(52) Cooperative Patent Classification (CPC):
**A61M 25/00; Y02P 10/25**

(86) International application number:
**PCT/CN2021/132607**

(87) International publication number:
**WO 2022/127536 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.12.2020 CN 202011496765**

(71) Applicant: **MicroPort NeuroTech (Shanghai) Co., Ltd.**
**Shanghai 201318 (CN)**

(72) Inventors:
• **LIU, Yunyun**
  **Shanghai 201318 (CN)**
• **LIU, Qinglong**
  **Shanghai 201318 (CN)**
• **LUO, Xueli**
  **Shanghai 201318 (CN)**
• **LIU, Yumei**
  **Shanghai 201318 (CN)**

(74) Representative: **Marks & Clerk LLP**
**15 Fetter Lane**
**London EC4A 1BW (GB)**

(54) **MEDICAL CATHETER AND PREPARATION METHOD THEREFOR**

(57) A medical catheter and a method for fabricating it are disclosed. The medical catheter includes a catheter body (100) including a proximal section (1) and a distal section (2) sequentially arranged along an axis. Stiffness of the proximal section (1) is greater than stiffness of the distal section (2). The distal section (2) includes a transition section (21) and a flexible section (22) from a proximal end to a distal end thereof. Stiffness of the flexible section (22) is smaller than stiffness of the transition section (21). The stiffness of the transition section (21) gradually decreases from a proximal end to a distal end thereof. A ratio of an axial length of the flexible section (22) to an axial length of the transition section (21) is 0.05-0.4. This axial stiffness design of the medical catheter can achieve a balance between its accessibility and support performance, which can result in better therapeutic outcomes of a surgical procedure.

Fig. 1

EP 4 249 030 A1

**Description**

**TECHNICAL FIELD**

The present invention relates to the field of medical devices and, in particular, to a medical catheter and a method for fabricating it.

**BACKGROUND**

As crucial devices in the field of interventional therapy, catheters are usually inserted into a biological lumen such as a blood vessel and advanced through the biological lumen to access a lesion site in need of treatment or diagnosis. When accessing a lesion site, the catheters are used to deliver a medical device, medicament or draw an occluding substance therefrom.

Given this mode of action, a catheter must be able to be advanced within a biological lumen in a satisfactory way while not causing damage to the biological lumen. In endovascular interventional procedures, a catheter is usually introduced into a human body through the radial artery or the femoral artery. In some procedures, in particular intracranial vascular procedures, a catheter is required to be advanced through tortuous blood vessels to reach a distal location and therefore must have good accessibility. The accessibility of a catheter is generally related to its flexibility, and a more flexible catheter tends to be advanced through a tortuous blood vessel more easily. Moreover, when a catheter reaches a lesion site, a medical device or a medicament will be delivered therethrough, or an occluding substance will be drawn therethrough. In order to ensure successful delivery of the medical device or medicament or drawing of the occluding substance, the catheter is desired to have good ability to stay stationary without displacement under the action of the delivery or drawing. That is, the catheter is desired to have good support performance. The support performance of a catheter is generally related to its stiffness, and a catheter with appropriate stiffness can desirably remain stationary during passage of a medical device or the other therethrough.

To sum up, a catheter is desired to have good flexibility, which can impart good accessibility to the catheter. Moreover, the catheter is also desired to have appropriate stiffness, which can impart good support performance to the catheter. Accordingly, a catheter is generally designed as a structure, which is stiff around its proximal end and flexible around its distal end. However, the flexible distal portion is unfavorable to the catheter's support performance, and the stiff proximal portion is inconducive to the catheter's accessibility. There is no conventional catheter with well-balanced accessibility and support performance.

**SUMMARY OF THE INVENTION**

It is an object of the present invention to provide a medical catheter and a method for fabricating the catheter, which overcome the problem of unbalanced accessibility and support performance associated with the existing catheters.

To this end, the medical catheter provided in the present invention comprises a catheter body, the catheter body comprising a proximal section and a distal section sequentially arranged along an axis, the proximal section having stiffness that is greater than stiffness of the distal section, the distal section comprising a transition section and a flexible section sequentially arranged from a proximal end to a distal end thereof, the flexible section having stiffness that is smaller than stiffness of the transition section, the stiffness of the transition section gradually decreasing from a proximal end to a distal end thereof, a ratio of an axial length of the flexible section to an axial length of the transition section being 0.05-0.4.

Optionally, in the medical catheter, the ratio of the axial length of the flexible section to the axial length of the transition section may be 0.1-0.25.

Optionally, the catheter body may further comprise a tip section located distally with respect to the distal section, the tip section having stiffness that is smaller than the stiffness of the proximal section, the tip section having an axial length ranging from 0.2 cm to 6 cm.

Optionally, the tip section may comprise at least one radiopaque marker, which is any one of a radiopaque ring, a radiopaque band, a radiopaque spring and a radiopaque dot, or a combination thereof.

Optionally, in the medical catheter, a ratio of a two-point bending flexural strength of the flexible section to a two-point bending flexural strength of the proximal section may be 0.005-0.7, and/or the two-point bending flexural strength of the flexible section may range from 0.01 N to 1.0 N.

Optionally, in the medical catheter, the axial length of the transition section may range from 18 cm to 33 cm, and the axial length of the flexible section may range from 1.2 cm to 10 cm.

Optionally, in the medical catheter, the distal section may have an axial length ranging from 25 cm to 35 cm, and the catheter body may have a total axial length ranging from 100 cm to 160 cm.

Optionally, the medical catheter may comprise a radially outer layer, a radially intermediate layer and a radially inner layer, each of the outer layer and the inner layer made of a polymeric material, the intermediate layer being a braided structure and/or a spiral structure.

Optionally, in the medical catheter, in case of the intermediate layer being a braided structure, a braid density of a distal end of the intermediate layer may be lower or higher than a braid density of a proximal end of the intermediate layer, or in case of the intermediate layer being a spiral structure, the intermediate layer may have a spiral density gradually decreasing from a proximal end to a distal end thereof.

Optionally, in the medical catheter, the intermediate layer may be spirally wound from single-stranded wire, or the intermediate layer may be braided and/or spirally wound from multi-stranded wires.

Optionally, in the medical catheter, stiffness of the polymeric material of the outer layer may gradually decease from a proximal end to a distal end thereof, and/or the outer layer may have a wall thickness gradually deceasing from the proximal end to the distal end thereof and have a constant inner diameter.

Optionally, the medical catheter may have an inner diameter ranging from 0.40 mm to 2.24 mm and an outer diameter ranging from 0.42 mm to 2.8 mm.

To the above end, the method provided in the present invention comprises:

providing a catheter body comprising a proximal section and a distal section sequentially arranged along an axis, and wherein stiffness of the proximal section is greater than stiffness of the distal section;

providing the distal section with a transition section and a flexible section sequentially arranged from a proximal end to a distal end thereof, wherein stiffness of the flexible section is smaller than stiffness of the transition section, and the stiffness of the transition section gradually decreases from a proximal end to a distal end thereof; and

configuring a ratio of an axial length of the flexible section to an axial length of the transition section 21 in the range of 0.05-0.4.

Optionally, in the method, the ratio of the axial length of the flexible section to the axial length of the transition section may be 0.1-0.25.

Optionally, in the method, two-point bending flexural strength of the flexible section and two-point bending flexural strength of the proximal section may satisfy

$$\frac{F2}{F1} \in [0.005, 0.7]$$

and/or

$$F2 \in [0.01\ \text{N}, 1.0\ \text{N}],$$

where F2 represents the two-point bending flexural strength of the flexible section, measured in N, and F1 represents the two-point bending flexural strength of the proximal section, measured in N.

Optionally, in the method, the axial length of the transition section may range from 18 cm to 33 cm, and the axial length of the flexible section may range from 1.2 cm to 10 cm.

Optionally, in the method, the distal section may have an axial length ranging from 25 cm to 35 cm, and the catheter body may have a total axial length ranging from 100 cm to 160 cm.

Optionally, the method may further comprise:

providing the catheter body with a radially outer layer, a radially intermediate layer and a radially inner layer, wherein each of the outer layer and the inner layer is made of a polymeric material, the intermediate layer being a braided structure and/or spiral structure.

In the above-described medical catheter, the catheter body is configured to include the proximal section and the distal section, and the stiffness of the proximal section is greater than the stiffness of the distal section. In particular, the distal section includes the transition section and the flexible section, and the stiffness of the flexible section is smaller than the stiffness of the transition section. Moreover, the stiffness of the transition section gradually decreases from the proximal end to the distal end thereof. In particular, the ratio of the axial length of the flexible section to the axial length of the transition section ranges from 0.05 to 0.4. With this configuration, a balance can be achieved between support performance and accessibility, and hence between support performance and the ability to pass through bends, of the medical catheter, through properly configuration the length and stiffness of the distal section. As a result, the medical catheter can pass through tortuous blood vessels and is able to stay stationary as much as possible at a desired location with minimal displacement when a medical device or another object is being delivered in lumen of the medical catheter.

Therefore, in a surgical procedure using the medical catheter, the medical catheter can reach a relatively distant lesion position without displacement under the effect of a therapeutic action. This can improve therapeutic outcomes of the procedure and reduce the time that the procedure requires.

More specifically, if the distal section is excessively long, the medical catheter will have good accessibility, but its support performance will be poor. On the contrary, if the distal section is too short, the medical catheter will have good support performance, but its accessibility will be poor. In order to achieve a balance between support performance and accessibility of the medical catheter, and hence between its support performance and accessibility, it is necessary to properly configure the length and stiffness of the distal section in medical catheter. Moreover, the axial length ratio of the transition section to the flexible section in the distal section should not be too large or too small. If the ratio is excessively large, the length of the transition section will be too short. Consequently, flexibility at the distal end of the catheter cannot reach an appropriate level, leading to poor accessibility of the catheter. Alternatively, even when the catheter has an appropriate level of flexibility at the distal end, due to an abrupt transition in flexibility/stiffness, the transition section will have poor kink resistance. If the ratio is too small, the flexible section will have an excessively short length. This will raise the complexity and cost of fabrication, and the short flexible section will lead to poor ability of the catheter head to pass through bends.

Through configuring the two-point bending flexural strength ratio of the flexible section to the proximal section in the medical catheter within the aforementioned predetermined range, the medical catheter is enabled to overall have good transitioning between flexible and stiff portions, which additionally reduce resistance that the catheter encounters, as well as the risk of the catheter being kink, when it is being delivered, thereby reducing the complexity and required time of a surgical procedure using the medical catheter. In addition, through configuring the two-point bending flexural strength of the flexible section within the aforementioned predetermined range, the distal potion of the medical catheter will have such appropriate flexibility that it will not be difficult to bend, or will not easily cause damage to a blood vessel, due to excessively high stiffness. Moreover, it will not affect the support performance and force transmission due to excessively high flexibility.

## BRIEF DESCRIPTION OF THE DRAWINGS

Those of ordinary skill in the art would appreciate that the accompanying drawings are provided to facilitate a better understanding of the present invention and do not limit the scope thereof in any sense, in which:

Fig. 1 is a schematic diagram showing the overall structure of a medical catheter according to a preferred embodiment of the present invention;
Fig. 2 is an axial cross-sectional view of a medical catheter according to a preferred embodiment of the present invention;
Fig. 3 is a transverse cross-sectional view of a medical catheter according to a preferred embodiment of the present invention;
Fig. 4 is a schematic illustration of a distal section according to a preferred embodiment of the present invention; and
Fig. 5 is an axial cross-sectional view of an outer layer according to a preferred embodiment of the present invention.

In these figures,

100, a catheter body;
1, a proximal section; 2, a distal section; 3, a tip section; 21, a transition section; 22, a flexible section; L22, an axial length of the flexible section; L21, an axial length of the transition section; 101, an outer layer; 102, an intermediate layer; 103, an inner layer; 101a, a first segment of the outer layer; 101b, a second segment of the outer layer; 101c, a third segment of the outer layer; L1, an axial length of the first segment of the outer layer; L2, an axial length of the second segment of the outer layer; L3, an axial length of the third segment of the outer layer; 200, a stress diffusion tube; 300, a proximal connecting member; and 104, a radiopaque marker.

Throughout the several views, similar numerals indicate similar elements.

## DETAILED DESCRIPTION

Objects, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments.

As used herein, the singular forms "a", "an" and "the" include plural referents, unless the context clearly dictates otherwise. As used herein and in the appended claims, the term "or" is generally employed in the sense of "and/or", "plurality" of is generally employed in the sense of "two or more", and "several" of is generally employed in the sense of "an indefinite number of', unless the context clearly dictates otherwise. As used herein, the term "proximal end" generally refers to an end closer to an operator who is operating a medical device, and the term "distal end" generally refers to an end of the device that enters the body of a patient first, unless the context clearly dictates otherwise.

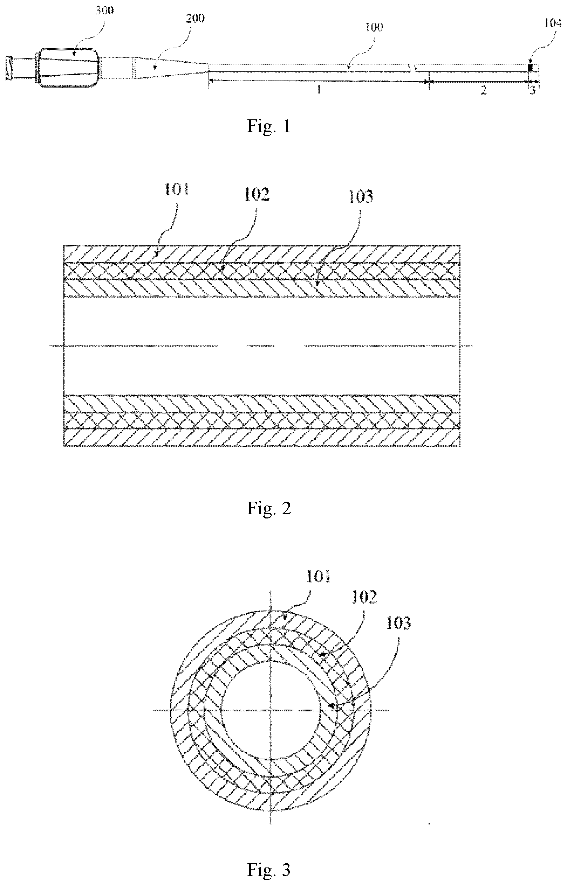

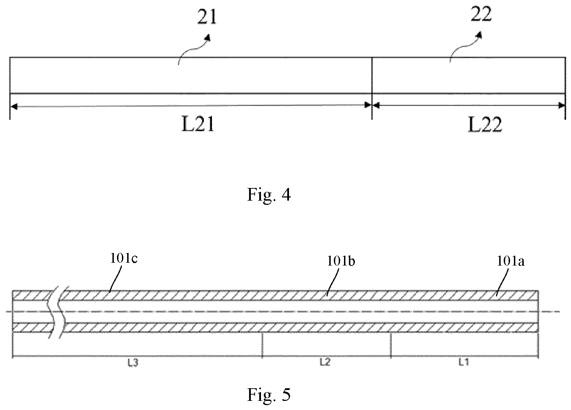

Fig. 1 is a schematic diagram showing the overall structure of a medical catheter according to a preferred embodiment of the present invention. Fig. 2 is an axial cross-sectional view of a medical catheter according to a preferred embodiment of the present invention. Fig. 3 is a transverse cross-sectional view of a medical catheter according to a preferred embodiment of the present invention, taken along a transverse plane that is perpendicular to an axis of the medical catheter. Fig. 4 is a schematic illustration of a distal section according to a preferred embodiment of the present invention. Fig. 5 is an axial cross-sectional view of an outer layer according to a preferred embodiment of the present invention.

As shown in Fig. 1, in an embodiment of the present invention, there is provided a medical catheter, which includes a catheter body 100 including a proximal section 1 and a distal section 2 sequentially arranged along an axially. The catheter body 100 further includes a tip section 3 located distally with respect to the distal section 2. The proximal section 1 is stiffer than each of the distal section 2 and the tip section 3. The inventors have found that a length and stiffness of the distal section 2 have marked impacts on support performance and accessibility (i.e., ability to pass through bends) of the medical catheter. If the distal section 2 is excessively long, the medical catheter will have good accessibility, but its support performance will be poor. On the contrary, if the distal section 2 is too short, the medical catheter will have good support performance, but its accessibility will be poor. In order to achieve a balance between support performance and accessibility of the medical catheter, it is necessary to reasonably control the length and stiffness of the distal section 2 in the medical catheter.

As shown in Fig. 4, the distal section 2 includes a transition section 21 and a flexible section 22 arranged axially from the proximal end to the distal end thereof. Stiffness of the flexible section 22 is lower than that of the transition section 21. The stiffness of the transition section 21 gradually decreases from a proximal end to a distal end thereof. As a part of the catheter, the gradually decreasing stiffness of the transition section 21 imparts to the catheter stiffness that transitions from the stiffness of the proximal section 1 to the stiffness of the flexible section 22. Here, by "gradually decreasing stiffness", it is intended to mean that the stiffness may decrease, then remain constant and then further decrease, or may decrease continually, or may decrease otherwise. That is, the present invention is not limited to any particular manner in which the stiffness decreases, as long as it is ensured that the stiffness overall decreases from the proximal end to the distal end. A ratio of an axial length L22 of the flexible section 22 to an axial length L21 of the transition section 21 is 0.05-0.4, more preferably 0.1-0.25, such as 0.05, 0.08, 0.1, 0.15, 0.2, 0.25, 0.3, 0.35 or 0.4. With this design, a balance can be achieved between support performance and ability to pass through bends of the medical catheter through properly configuring the length and stiffness of the distal section 2. As a result, the medical catheter can be advanced through tortuous blood vessels, while being able to remain stationary as much as possible with minimized displacement when a medical device or another object is being delivered in a lumen of the medical catheter. Thus, during a procedure, the medical catheter is able to reach a relatively distant lesion site and will not displace under the effect of a therapeutic action. This ensures desirable therapeutic outcomes and can shorten the time required by a procedure using the catheter. More importantly, through properly controlling the ratio of the axial length L22 of the flexible section 22 to the axial length L21 of the transition section 21, an even better balance can be achieved between support performance and accessibility of the catheter. If the ratio is excessively large, the axial length of the transition section 21 will be too short. Consequently, the flexibility at the distal end of the catheter cannot reach an appropriate level, leading to poor accessibility of the catheter. Alternatively, even when the catheter has an appropriate level of flexibility at the distal end, due to an abrupt transition in flexibility/stiffness, the transition section 21 will have poor kink resistance. If the ratio is too small, the distal flexible section 22 will have an excessively short axial length. This will raise the complexity and cost of fabrication, and the short flexible section 22 will lead to poor ability of the catheter head to pass through bends.

The axial length L21 of the transition section 21 is in the range of from 18 cm to 33 cm, such as 18 cm, 20 cm, 25 cm, 28 cm, 30 cm, 33 cm or the like. The axial length L22 of the flexible section 22 is in the range of from 1.2 cm to 10 cm, such as 1.2 cm, 1.5 cm, 2 cm, 3 cm, 5 cm, 8 cm, 10 cm or the like. The axial length of the distal section 2 is in the range of from 25 cm to 35 cm, preferably 28 cm to 32 cm, such as 28 cm, 29 cm, 30 cm, 32 cm or the like. The tip section 3 has an axial length in the range of from 0.2 cm to 6.0 cm, preferably 1 cm to 3 cm, such as 2 cm, 1.5 cm, 1.8 cm, 2 cm, 3 cm or the like. The catheter body 110 has total axial length in the range of from 100 cm to 160 cm, preferably 105 cm to 150 cm, such as 115 cm, 120 cm, 125 cm, 150 cm or the like. Implementations of the medical catheter of the present invention include, but are not limited to, those for intracranial use. Moreover, it is not limited to being used to deliver a medical device or a medicament, and can also be used to draw out and remove an occluding substance or perform another suitable task.

The catheter body 100 has an inner diameter in the range of 0.40 mm to 2.24 mm, such as 0.4 mm, 1 mm, 1.2 mm, 1.8 mm, 2 mm, 2.24 mm or the like. The catheter body 100 has an outer diameter in the range of 0.42 mm to 2.8 mm, such as 0.42 mm, 0.8 mm, 1.2 mm, 1.5 mm, 1.8 mm, 2 mm, 2.5 mm, 2.8 mm or the like. Preferably, the inner diameter of the catheter body 100 is constant, while the outer diameter decreases from the proximal end to the distal end. Accordingly, the medical catheter has a varying wall thickness, which is smaller at the distal end and greater at the proximal end. This can further enhance the support performance of the medical catheter at the proximal end and the ability to pass through bends at the distal end. For example, the outer diameter of the catheter at the proximal end is 2.12 mm and 2.08 mm at the distal end.

As shown in Figs. 2 and 3, the catheter body 100 preferably includes a radially outer layer 101, a radially intermediate layer 102 and a radially inner layer 103, which are arranged in coaxiality with one another. Both the outer layer 101 and the inner layer 103 are formed of polymeric materials. The intermediate layer 102 is a reinforcing layer, which may be a braided and/or spiral structure and functions to increase support strength of the catheter, enhance its axial force transmission and improve its kink resistance. The intermediate layer 102 may be formed of braided and/or spirally wound metal or polymer wires. The inner layer 103 may be formed of any one of polytrafluoroethylene, polyolefin, polyether block amide and polyurethane, or a combination thereof. The intermediate layer 102 may be formed of metal wires, or polymer wires, or both. The outer layer 101 may be made of at least one of polyamide, Pebax, polyurethane and polyolefin. The inner layer 103 is preferably provided with a hydrophilic coating or a hydrophobic coating on its inner surface, which can reduce a coefficient of friction of the lumen of the medical catheter or frictional resistance of the inner surface. Further, the intermediate layer 102 may have a braid density at its distal end that is lower or higher than a braid density at a proximal end thereof. Alternatively, the intermediate layer 102 may have a spiral density decreasing from the proximal end to the distal end. Such a braid or spiral density arrangement enables stiffness control of the intermediate layer 102 for additionally modifying the support performance and accessibility of the catheter.

In one embodiment, the intermediate layer 102 is a braided structure. Moreover, the medical catheter may have different braid densities (measured in pitches per inch (PPI), i.e., the number of braid intersections in each inch of axial length) at different portions(e.g., the position of transition section) thereof. For example, depending on PPI, the intermediate layer 102 may include n1 segments, which are joined together axially, where n1 may range from 1 to 10. Distal segment may have a lower braid density, and proximal segment may have a higher braid density. Alternatively, proximal segment may have a lower braid density, and distal segment may have a higher braid density. Still alternatively, appropriately increased braid densities may be present at transition locations of the outer layer 101. The braid density of the intermediate layer 102 may range from 40 PPI to 300 PPI.

In another embodiment, the intermediate layer 102 is a spiral structure with a spiral density (measured in pitches per inch (PPI), i.e., the number of spirals in each inch of axial length) decreasing from the proximal end to the distal end thereof. Similarly, the spiral density of the spiral structure may range from 40 PPI to 300 PPI. For example, the spiral density may be 60 PPI at the proximal end and 125 PPI at the distal end. Additionally, in case of the intermediate layer 102 being implemented as a spiral structure, the medical catheter may have different spiral densities at different portions thereof. For example, depending on PPI, the intermediate layer 102 may include n1 segments, which are joined together axially, where n1 may range from 1 to 10.

The intermediate layer 102 may be braided and/or spirally wound from one or more of double-stranded round wires, double-stranded flat wires, single-stranded round wires, single-stranded flat wires, multi-stranded round wires and multi-stranded flat wires. The wires in the intermediate layer 102 may be nickel-titanium, stainless steel, cobalt-chromium, polymer or other wires for medical use preferably having a diameter of 0.0005-0.03 inches. In some embodiments, the intermediate layer 102 may be braided from 4-64 wires in a "two on two" or "one on one" style. In some other embodiments, the intermediate layer 102 may be a structure made up of one or more wires spirally wound at an angle of 5-90° with respect to an axis of the catheter. In one preferred embodiment, the intermediate layer 102 is a spring structure made of a single-stranded flat stainless steel or nickel-titanium alloy wire with a width of 0.006 inches and a thickness of 0.002 inches. In another preferred embodiment, the intermediate layer 102 is a spring structure made of a single-stranded round stainless steel or nickel-titanium alloy wire with a diameter of 0.01 inch.

In order to additionally enhance flexibility at the distal end of the catheter while ensuring satisfactory support performance at the proximal end of the catheter, the outer layer 101 is preferably designed with stiffness decreasing from the proximal end to the distal end. In some embodiments, the outer layer 101 is made of a polymeric material with stiffness decreasing from the proximal end to the distal end. In other embodiments, the outer layer 101 may have a wall thickness decreasing from the proximal end to the distal end while maintaining a constant inner diameter across its entire length. More particularly, the outer layer 101 may consist of different segments joined together, which are made of the same polymeric material but have different levels of stiffness, or which are made of different polymeric materials and have different levels of stiffness. That is, the outer layer 101 may be a structure consisting of multiple polymeric segments which are joined together and have different levels of stiffness. In this case, the outer layer 101 may include n2 segments, which are axially joined together, where n2 may range from 3 to 20. Overall stiffness of the n2 segments may decrease from the proximal end to the distal end. As an example, as shown in Fig. 5, the outer layer 101 may have three segments with overall stiffness increasing from the distal end to the proximal end. Specifically, the three segments may be a first segment 101a, a second segment 101b and a third segment 101c sequentially arranged from the distal end to the proximal end thereof. The first segment 101a may have the lowest stiffness, and the third segment 101c may have the highest stiffness. The stiffness of the second segment 101b may lie between the stiffness of the first segment 101a and the stiffness of the third segment 101c.

In another embodiment, the outer layer 101 may consist of segments joined together, which are made of different polymeric materials with different levels of stiffness. That is, the outer layer 101 is a structure consisting of multiple segments joined together. In this case, likewise, the outer layer 101 may include n2 segments, which are axially joined together, where n2 may range from 3 to 20, and overall stiffness of the n2 segments may decrease from the proximal end to the distal end. For example, the first segment 101a may be a polyurethane segment having an axial length L1; the second segment 101b may be a Pebax segment having an axial length L2; and the third segment 101c may be a polyamide segment having an axial length L3.

The axial length L1 of the first segment 101a may range from 30 mm to 150 mm. The axial length L2 of the second segment 101b may range from 200 mm to 500 mm. The axial length L3 of the third segment 101c may range from 700 mm to 1200 mm. The inner diameter of the outer layer 101 may range from 0.021 inches to 0.10 inches, and the outer diameter thereof may range from 0.03 inches to 0.11 inches (0.76 mm to 2.8 mm).

It would be appreciated that the varying stiffness of the outer layer 101 facilitates the formation of a stiffness profile of the medical catheter gradually decreasing from the proximal end to the distal end, which increases flexibility of the medical catheter at the distal end and additionally guarantees delivery performance and safety of the medical catheter.

With continued reference to Fig. 1, the medical catheter may further include a stress diffusion tube 200 and a proximal connecting member 300. The stress diffusion tube 200 may be coupled to the proximal end of the catheter body 100. The proximal connecting member 300 may be provided thereon with various interfaces such as fluid inlet interfaces, lumen interfaces and the like.

With continued reference to Fig. 1, the tip section 3 of the medical catheter may include a radiopaque marker 104 optionally implemented as any one of a radiopaque ring, a radiopaque band, a radiopaque spring and a radiopaque dot, or any combination thereof. The radiopaque marker 104 may be made of a radiopaque metal such as platinum, tungsten or gold, or a polymeric material mixed therein with a radiopaque metal powder. In one preferred embodiment, the radiopaque marker 104 is a radiopaque ring. The radiopaque marker 104 serves to mark the position of the catheter head, enabling a surgeon to visually monitor the position and condition of the catheter during a surgical procedure and accordingly make adjustments to a delivery path and stress condition of the catheter.

In order to further improve the performance of the medical catheter, a ratio of two-point bending flexural strength of the flexible section 22 to two-point bending flexural strength of the proximal section 1 is preferably designed to satisfy:

$$\frac{F2}{F1} \in [\,0.005, 0.7\,]$$

where F2 represents the two-point bending flexural strength of the flexible section 22, measured in N, and F1 represents the two-point bending flexural strength of the proximal section 1, measured in N.

Such a ratio enables the medical catheter to overall have good transitioning between flexible and stiff portions, which additionally reduce resistance that the catheter encounters, as well as the risk of the catheter being kinked, when it is being delivered.

Additionally, the two-point bending flexural strength of the flexible section 22 preferably satisfies:

$$F2 \in [\,0.01\ \text{N}, 1.0\ \text{N}\,]$$

When the two-point bending flexural strength F2 of the flexible section 22 is designed within this range, the distal section of the medical catheter will have such appropriate flexibility that it will not be difficult to bend, or will not easily cause damage to a blood vessel, due to high stiffness. Moreover, it will not affect the support performance and force transmission due to high flexibility.

In embodiments of the present invention, there is also provided a method for fabricating a medical catheter, which includes:

providing a catheter body 100 and configuring the catheter body 100 to include a proximal section 1 and a distal section 2 sequentially arranged along the axis, and further to include a tip section 3, the stiffness of the proximal section 1 is higher than the stiffness of each of the distal section 2 and stiffness of the tip section 3;
providing the distal section 2 with a transition section 21 and a flexible section 22 sequentially arranged from the proximal end to the distal end, the stiffness of the flexible section 22 is lower than stiffness of the transition section 21, and the stiffness of the transition section 21 gradually decreases from a proximal end to a distal end thereof; and configuring a ratio of an axial length of the flexible section 22 to an axial length of the transition section 21 in the range of 0.05-0.4.

The method may further include:

providing the catheter body 100 with a radially outer layer 101, a radially intermediate layer 102 and a radially inner layer 103. Both the outer layer 101 and the inner layer 103 may be made of polymeric materials, and the intermediate layer 102 may be a braided and/or spiral structure.

Advantages of the medical catheter of the present invention will be further explained with reference to specific test data. However, it would be appreciated that parameters used in such tests are not intended to impose any limitation on the structure of the medical catheter of the present invention.

In the present embodiment, the two-point bending flexural strength of the proximal section 1 in the medical catheter is denoted as F1, and the two-point bending flexural strength of the flexible section 22 in the distal section 2 as F2. In order to enable better force transmission in the catheter body, F2/F1 may range from 0.005 to 0.7, and F2 may range from 0.01 N to 1.0 N.

It would be appreciated that two-point bending flexural strength is a metric for assessing flexibility of the medical catheter, and higher two-point bending flexural strength indicates better flexibility of the medical catheter. Two-point bending flexural strength may be measured using a method in which about 2-cm pieces are cut off from each test catheter sample (respectively from the proximal and flexible sections), and a proximal portion of each piece is inserted into a chuck of a lower jaw, leaving the remaining distal portion of the piece outside of the chuck. After the chuck is tightened, a length of 10 mm to 15 mm of the piece is exposed. The jig is manipulated to lower a mechanical sensor until it just comes into contact with the piece. The piece is then adjusted so that its distal end fits over the pressure head of mechanical sensor, but no force is present therebetween. A downward pressing stroke d is set as 2.5 mm and a pressing speed as 2.5 mm/min in an associated program, and maximum downward pressure N1 is recorded. The test conditions and results are shown in Table 1.

Table 1 Design parameters of test samples and comparative samples

| Sample | Axial Length of Proximal Section | Axial Length L21 of Transition Section | Axial Length L22 of Flexible Section | L22/L21 | Two-Point Flexural Strength F2 of Flexible Section (N) | Two-Point Flexural Strength F1 of Proximal Section (N) |
|---|---|---|---|---|---|---|
| Test Sample 1 | 100 cm | 25 cm | 5 cm | 0.2 | 0.1 | 1.4 |
| Test Sample 2 | 100 cm | 24 cm | 8 cm | 0.33 | 0.1 | 1.4 |
| Test Sample 3 | 100 cm | 22 cm | 3 cm | 0.14 | 0.1 | 1.4 |
| Test Sample 4 | 100 cm | 33 cm | 2 cm | 0.06 | 0.1 | 1.4 |
| Comparative Sample 1 | 100 cm | 15 cm | 5 cm | 0.33 | 0.1 | 1.4 |
| Comparative Sample 2 | 100 cm | 11 cm | 19 cm | 1.72 | 0.1 | 1.4 |
| Comparative Sample 3 | 100 cm | 13 cm | 17 cm | 1.31 | 0.1 | 1.4 |
| Comparative Sample 4 | 100 cm | 12 cm | 8 cm | 0.66 | 0.1 | 1.4 |
| Comparative Sample 5 | 100 cm | 30 cm | 12 cm | 0.4 | 0.1 | 1.4 |

Four test samples and five comparative samples were tested, and the two-point bending flexural strength F2 of the flexible section was measured as 0.1 N and the two-point bending flexural strength F1 of the proximal section 1 as 1.4 N. Thus, F22/F1≈0.071.

Additionally, resistance to advancement and support performance of the four test samples and five comparative samples were tested on 3D cerebrovascular model. Maximum resistance to advancement (measured in gf) was measured as the maximum resistance that the sample encountered during its advancement to a site M2 (the insular segment of the middle cerebral artery) in an intracranial blood vessel in the cerebrovascular model. Support performance was tested by fixing the catheter sample at a location in the model and passing a stent through the catheter sample. A distance that the catheter head retracted was observed after the stent is pushed over a predetermined distance. A smaller distance that the catheter head retracted indicated better ability of the catheter to remain stationary at a desired location. The final test results are summarized in Table 2.

Table 2 Performance test results of test samples and comparative samples

| Sample | Maximum Resistance to Advancement (gf) | Retraction Distance of Catheter Head Caused by Passage of Stent (mm) |
|---|---|---|
| Test Sample 1 | 60.2 | 2 |
| Test Sample 2 | 55.8 | 3 |
| Test Sample 3 | 69.3 | 2 |
| Test Sample 4 | 53.5 | 4 |
| Comparative Sample 1 | 101.3 | 2 |
| Comparative Sample 2 | 68.6 | 15 |
| Comparative Sample 3 | 62.2 | 14 |
| Comparative Sample 4 | 98.6 | 2 |
| Comparative Sample 5 | 70.3 | 11 |

As can be seen from Table 2, the 4 test samples encountered significantly reduced resistance to advancement and exhibited greatly improved support performance, compared to the 5 comparative samples. Comparative Samples 1 and 4 encountered much greater resistance to advancement, and Comparative Samples 2 and 3 showed very poor support performance due to an excessively large axial length ratio of the flexible section to the transition section. Although Comparative Sample 5 encountered low resistance to advancement, its support performance was unsatisfactory. Apparently, all the 4 test samples are superior in terms of both resistance to advancement and support performance. Therefore, the design of the test samples (i.e., medical catheter prepared in accordance with the present invention) achieves a good balance between support performance and ability to pass through bends, which profoundly benefits the catheters.

The description presented above is merely that of some preferred embodiments of the present invention and does not limit the scope thereof in any sense. Any and all changes and modifications made by those of ordinary skill in the art based on the above teachings are intended to fall within the scope of the present invention.

**Claims**

1. A medical catheter, comprising a catheter body, wherein the catheter body comprising a proximal section and a distal section sequentially arranged along an axis, wherein the proximal section has a stiffness that is greater than a stiffness of the distal section, wherein the distal section comprises a transition section and a flexible section sequentially arranged from a proximal end to a distal end thereof, wherein the flexible section has a stiffness that is smaller than a stiffness of the transition section, wherein the stiffness of the transition section gradually decreases from a proximal end to a distal end thereof, and wherein a ratio of an axial length of the flexible section to an axial length of the transition section is 0.05-0.4.

2. The medical catheter according to claim 1, wherein the ratio of the axial length of the flexible section to the axial length of the transition section is 0.1-0.25.

3. The medical catheter according to claim 1, wherein the catheter body further comprises a tip section located distally with respect to the distal section, wherein the tip section has a stiffness that is smaller than the stiffness of the proximal section, and wherein the tip section has an axial length ranging from 0.2 cm to 6 cm.

4.   The medical catheter according to claim 3, wherein the tip section comprises at least one radiopaque marker, and wherein the radiopaque marker is any one of a radiopaque ring, a radiopaque band, a radiopaque spring and a radiopaque dot, or a combination thereof.

5.   The medical catheter according to claim 1, wherein a ratio of a two-point bending flexural strength of the flexible section to a two-point bending flexural strength of the proximal section is 0.005-0.7, and/or wherein the two-point bending flexural strength of the flexible section ranges from 0.01 N to 1.0 N.

6.   The medical catheter according to claim 1, wherein the axial length of the transition section ranges from 18 cm to 33 cm, and wherein the axial length of the flexible section ranges from 1.2 cm to 10 cm.

7.   The medical catheter according to claim 1, wherein the distal section has an axial length ranging from 25 cm to 35 cm, and wherein the catheter body has a total axial length ranging from 100 cm to 160 cm.

8.   The medical catheter according to any one of claims 1 to 7, comprising a radially outer layer, a radially intermediate layer and a radially inner layer, wherein each of the outer layer and the inner layer is made of a polymeric material, and wherein the intermediate layer is a braided structure and/or a spiral structure.

9.   The medical catheter according to claim 8, wherein in case of the intermediate layer being the braided structure, a braid density of a distal end of the intermediate layer is lower or higher than a braid density of a proximal end of the intermediate layer, or wherein in case of the intermediate layer being a spiral structure, the intermediate layer has a spiral density gradually decreasing from a proximal end to a distal end thereof.

10.  The medical catheter according to claim 8, wherein the intermediate layer is spirally wound from a single-stranded wire, or wherein the intermediate layer is braided and/or spirally wound from a multi-stranded wire.

11.  The medical catheter according to claim 8, wherein a stiffness of the polymeric material of the outer layer gradually deceases from a proximal end to a distal end thereof, and/or wherein the outer layer has a wall thickness gradually deceasing from the proximal end to the distal end thereof and has a constant inner diameter.

12.  The medical catheter according to any one of claims 1 to 7, wherein the medical catheter has an inner diameter ranging from 0.40 mm to 2.24 mm and an outer diameter ranging from 0.42 mm to 2.8 mm.

13.  A method for fabricating a medical catheter, comprising:

   providing a catheter body comprising a proximal section and a distal section sequentially arranged along an axis, wherein a stiffness of the proximal section is greater than a stiffness of the distal section;
   providing the distal section with a transition section and a flexible section sequentially arranged from a proximal end to a distal end thereof, wherein a stiffness of the flexible section is smaller than a stiffness of the transition section, and wherein the stiffness of the transition section gradually decreases from a proximal end to a distal end thereof; and
   configuring a ratio of an axial length of the flexible section to an axial length of the transition section in a range of 0.05-0.4.

14.  The method according to claim 13, wherein the ratio of the axial length of the flexible section to the axial length of the transition section is 0.1-0.25.

15.  The method according to claim 13, wherein a two-point bending flexural strength of the flexible section and a two-point bending flexural strength of the proximal section satisfy

$$\frac{F2}{F1} \in [\,0.005, 0.7\,]$$

and/or

$$F2 \in [\,0.01\ \text{N}, 1.0\ \text{N}],$$

where F2 represents the two-point bending flexural strength of the flexible section, measured in N; and F1 represents the two-point bending flexural strength of the proximal section, measured in N.

16. The method according to claim 13, wherein the axial length of the transition section ranges from 18 cm to 33 cm, and wherein the axial length of the flexible section ranges from 1.2 cm to 10 cm.

17. The method according to claim 13, wherein the distal section has an axial length ranging from 25 cm to 35 cm, and wherein the catheter body has a total axial length ranging from 100 cm to 160 cm.

18. The method according to any one of claims 13 to 17, further comprising:
providing the catheter body with a radially outer layer, a radially intermediate layer and a radially inner layer, wherein each of the outer layer and the inner layer is made of a polymeric material, and wherein the intermediate layer is a braided structure and/or a spiral structure.

Fig. 1

Fig. 2

Fig. 3

21 22

L21 L22

Fig. 4

101c 101b 101a

L3 L2 L1

Fig. 5

## INTERNATIONAL SEARCH REPORT

| International application No. |
|---|
| **PCT/CN2021/132607** |

| **A.** | **CLASSIFICATION OF SUBJECT MATTER** |
|---|---|

A61M 25/00(2006.01)i

According to International Patent Classification (IPC) or to both national classification and IPC

| **B.** | **FIELDS SEARCHED** |
|---|---|

Minimum documentation searched (classification system followed by classification symbols)

A61M

Documentation searched other than minimum documentation to the extent that such documents are included in the fields searched

Electronic data base consulted during the international search (name of data base and, where practicable, search terms used)

CNABS; CNTXT; VEN: 导管, 过渡, 段, 硬度, 逐渐, 柔软, 不同, 螺旋, 编织, 力, 弯曲力, catheter, microcatheter, flexibility, different, spiral, segment

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | CN 204364615 U (BEIJING GUOXIETANG TECHNOLOGY DEVELOPMENT CO., LTD.) 03 June 2015 (2015-06-03) description, paragraphs 5-21, and figures 1-2 | 1-8, 10-18 |
| Y | CN 204364615 U (BEIJING GUOXIETANG TECHNOLOGY DEVELOPMENT CO., LTD.) 03 June 2015 (2015-06-03) description, paragraphs 5-21, and figures 1-2 | 9 |
| X | US 2004153049 A1 (MICRO THERAPEUTICS INC.) 05 August 2004 (2004-08-05) description, paragraphs 29-74, and figures 1-9 | 1-18 |
| Y | US 2004153049 A1 (MICRO THERAPEUTICS INC.) 05 August 2004 (2004-08-05) description, paragraphs 29-74, and figures 1-9 | 9 |
| X | CN 101933821 A (MICROPORT MEDICAL SHANGHAI CO., LTD.) 05 January 2011 (2011-01-05) description, paragraphs 6-33, and figures 1-7 | 1-8, 10-18 |
| Y | CN 101933821 A (MICROPORT MEDICAL SHANGHAI CO., LTD.) 05 January 2011 (2011-01-05) description, paragraphs 6-33, and figures 1-7 | 9 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 February 2022** | **22 February 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| :--- |
| **PCT/CN2021/132607** |

**C.        DOCUMENTS CONSIDERED TO BE RELEVANT**

| Category* | Citation of document, with indication, where appropriate, of the relevant passages | Relevant to claim No. |
| :---: | :--- | :---: |
| X | CN 2889337 Y (MICROPORT MEDICAL SHANGHAI CO., LTD.) 18 April 2007 (2007-04-18) <br> description page 2 paragraph 2 to page 5 last paragraph, figures 1-3 | 1-8, 10-18 |
| Y | CN 2889337 Y (MICROPORT MEDICAL SHANGHAI CO., LTD.) 18 April 2007 (2007-04-18) <br> description page 2 paragraph 2 to page 5 last paragraph, figures 1-3 | 9 |
| Y | CN 108309383 A (SHANGHAI XINWEI MEDICAL TECHNOLOGY CO., LTD.) 24 July 2018 (2018-07-24) <br> description, paragraph 61 | 9 |
| Y | CN 201596219 U (BEIJING TAIJIE WEIYE TECHNOLOGY CO., LTD.) 06 October 2010 (2010-10-06) <br> description, paragraphs 26-30 | 9 |
| A | JP 2007029120 A (KANEKA CORPORATION) 08 February 2007 (2007-02-08) <br> entire document | 1-18 |

Form PCT/ISA/210 (second sheet) (January 2015)

**INTERNATIONAL SEARCH REPORT**
Information on patent family members

International application No.

**PCT/CN2021/132607**

| Patent document cited in search report | | | Publication date (day/month/year) | Patent family member(s) | | | Publication date (day/month/year) |
|---|---|---|---|---|---|---|---|
| CN | 204364615 | U | 03 June 2015 | None | | | |
| US | 2004153049 | A1 | 05 August 2004 | WO | 2004033015 | A1 | 22 April 2004 |
| | | | | ES | 2338001 | T3 | 03 May 2010 |
| | | | | EP | 1551489 | A1 | 13 July 2005 |
| | | | | EP | 1551489 | B1 | 09 December 2009 |
| | | | | US | 7507229 | B2 | 24 March 2009 |
| | | | | JP | 2011019983 | A | 03 February 2011 |
| | | | | JP | 5318073 | B2 | 16 October 2013 |
| | | | | JP | 2006501969 | A | 19 January 2006 |
| | | | | AT | 451135 | T | 15 December 2009 |
| | | | | AU | 2003277361 | A1 | 04 May 2004 |
| | | | | CA | 2501147 | A1 | 22 April 2004 |
| | | | | DE | 60330478 | D1 | 21 January 2010 |
| CN | 101933821 | A | 05 January 2011 | CN | 101933821 | B | 12 March 2014 |
| | | | | JP | 2012531224 | A | 10 December 2012 |
| | | | | EP | 2450077 | A1 | 09 May 2012 |
| | | | | EP | 2450077 | B1 | 24 October 2018 |
| | | | | KR | 20120106924 | A | 27 September 2012 |
| | | | | WO | 2011000264 | A1 | 06 January 2011 |
| CN | 2889337 | Y | 18 April 2007 | None | | | |
| CN | 108309383 | A | 24 July 2018 | CN | 208511116 | U | 19 February 2019 |
| CN | 201596219 | U | 06 October 2010 | None | | | |
| JP | 2007029120 | A | 08 February 2007 | None | | | |

Form PCT/ISA/210 (patent family annex) (January 2015)